# FASCICULE DE BREVET EUROPEEN

(11) **EP 4 052 670 B1**
(45) Date de publication et mention de la délivrance du brevet: **22.01.2025**
(21) Numéro de dépôt: 21315030.3
(22) Date de dépôt: 02.03.2021
(51) Int. Cl.: A61B 17/34

(54) **INSTRUMENT POUR SOUFFLER UN GAZ DANS UN ORGANE VIVANT POUR FAVORISER UNE DISSECTION MÉDICALE DE CET ORGANE**
INSTRUMENT ZUM EINBLASEN VON GAS IN EIN LEBENDES ORGAN ZUR ERLEICHTERUNG DER MEDIZINISCHEN SEZIERUNG DIESES ORGANS
INSTRUMENT FOR BLOWING A GAS INTO A LIVE ORGAN FOR AIDING A MEDICAL DISSECTION OF SAID ORGAN

(43) Date de publication de la demande: 07.09.2022
(73) Titulaire: AB Medica, 18100 Mery-sur-Cher (FR)
(72) Inventeur: Blanc, Alexandre, 13013 Marseille (FR)
(74) Mandataire: Flavenot, Bernard

(56) Documents cités:
- DD-A1- 234 608
- US-A1- 2009 030 438
- US-A1- 2017 209 165
- US-A1- 2018 344 329
- US-A1- 2021 045 722

## Description

La présente invention concerne les instruments pour souffler un gaz dans un organe vivant dans le but de favoriser une dissection médicale de cet organe, plus précisément les instruments qui permettent de délivrer un gaz sous pression pour séparer des couches de tissus d'êtres vivants dont au moins l'une doit subir une dissection, par exemple pour l'ablation d'une tumeur ou analogue.

On connaît déjà de tels instruments, ou instruments similaires, par exemple celui décrit et illustré dans le WO2016/012936 qui comporte essentiellement une poignée de manipulation apte à être saisie par le chirurgien, un orifice de sortie réalisée sur la poignée, des moyens d'alimentation en gaz, des moyens pour coupler ces moyens d'alimentation en gaz de façon que le gaz soit apte à être délivré sur l'orifice de sortie, un tube fin et oblong comportant une percée traversante définie entre une entrée proximale et une sortie distale, et des moyens pour connecter l'entrée proximale de la percée traversante avec l'orifice de sortie.

Sont aussi cités, comme art antérieur, les deux documents suivants : le US2018/344329 et le US2009/030438.

Avec un tel instrument qui est utilisé pour délivrer un gaz sous pression dans le but de séparer des couches de tissus vivants dont au moins l'une doit subir une dissection, il arrive assez fréquemment que la sortie distale de la percée traversante soit obstruée avec des caillots de sang ou analogues, ce qui empêche l'éjection du gaz pour obtenir la séparation optimale des couches de tissus vivants, et donc complique le travail du chirurgien.

La présente invention a pour but de réaliser un instrument qui tente de pallier en grande partie les inconvénients décrits ci-dessus des instruments similaires de l'art antérieur.

### Définition de l'invention

Plus précisément, la présente invention a pour objet un instrument pour souffler un gaz dans un organe vivant en vue de favoriser la dissection de cet organe. L'invention est définie par la revendication 1 et les revendications dépendantes divulguent des modes de réalisation.

### Brève Description des dessins

D'autres caractéristiques et avantages de la présente invention apparaîtront au cours de la description suivante donnée en regard des dessins annexés à titre illustratif, mais nullement limitatif, dans lesquels :
- La figure 1 est une vue de côté d'un mode de réalisation préféré de l'instrument selon l'invention pour souffler un gaz dans un organe vivant en vue de favoriser une dissection,
- La figure 2 est une vue en coupe longitudinale de la partie cerclée F2 de l'instrument selon la figure 1, et
- La figure 3 est une vue en coupe longitudinale de la partie cerclée F3 de l'instrument selon la figure 1.

Il est tout d'abord précisé que, sur l'ensemble des figures, les mêmes références désignent les mêmes éléments, quelle que soit la figure sur laquelle elles apparaissent et quelle que soit la forme de représentation de ces éléments. De même, si des éléments ne sont pas spécifiquement référencés sur l'une des figures, leurs références peuvent être aisément retrouvées en se reportant à une autre figure.

Il est de plus précisé que, dans la présente description, si l'adverbe "sensiblement" est associé à un qualificatif d'un moyen donné, ce qualificatif doit être compris au sens strict ou approché.

### Description d'un mode de réalisation préféré de l'objet de l'invention.

En référence aux figures annexées, la présente invention est relative à un instrument pour souffler un gaz dans un organe vivant pour favoriser la dissection d'au moins une partie de cet organe, comportant une poignée de manipulation 10 apte à être saisie par un chirurgien ou analogue, un orifice de sortie 11 réalisé sur la poignée, un tube 12 défini entre une extrémité proximale 114 et une extrémité distale 115 et comportant, sur toute sa longueur et selon son axe longitudinal, une percée traversante 13 définie entre une entrée proximale 14 et une sortie distale 15, des moyens pour connecter l'entrée proximale 14 de la percée traversante 13 avec l'orifice de sortie 11 de la poignée 10, et des moyens 16 pour coupler une source externe d'alimentation en gaz avec cet orifice de sortie 11.

Selon une caractéristique essentielle de l'invention, la portion de percée traversante qui est réalisée dans une partie Pb du tube 12 qui comporte l'extrémité distale 115 du dit tube, est évasée sensiblement en tronc de cône 20, optionnellement de révolution, la grande base 25 de ce tronc de cône étant sensiblement dans le plan de la sortie distale 15 de la percée traversante 13.

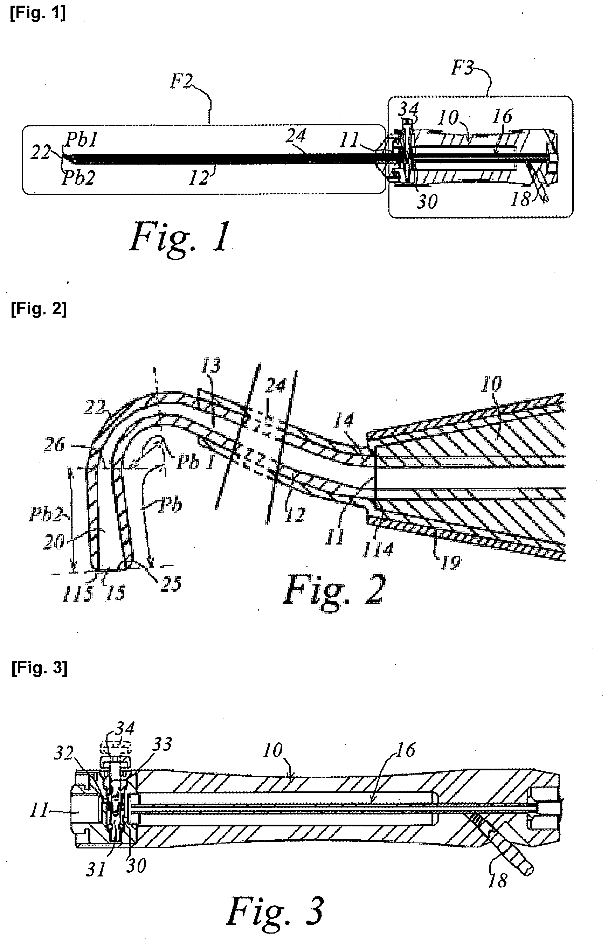

Selon une réalisation très préférentielle, qui présente des avantages très importants aussi bien sur le plan médical que sur le plan industriel notamment pour sa facilité de réalisation en fabrication, la partie Pb du tube 12 définie ci-dessus, figure 2, c'est-à-dire celle qui comporte l'extrémité distale 115 du tube, est conformée en crochet 22.

Ce crochet comporte une partie courbe Pb1 suivie d'une partie Pb2, celle qui comporte l'extrémité distale 115, sensiblement rectiligne.

Selon une réalisation très avantageuse, la portion de percée traversante 13 évasée en tronc de cône est réalisée dans la partie sensiblement rectiligne Pb2 du crochet 22, la petite base 26 du tronc de cône étant sensiblement située dans le plan qui sépare les parties du crochet Pb1, Pb2 respectivement courbe et rectiligne (Figure 2).

Selon une réalisation potentiellement préférée, la partie 24 du tube comprise entre l'orifice de sortie 11 réalisé sur la poignée 10 et la partie courbe Pb1 du crochet est cylindrique sensiblement rectiligne optionnellement de révolution.

Selon une réalisation préférentielle qui présente des avantages très importants sur le plan de la fabrication qu'elle simplifie, pour obtenir notamment une configuration de la percée traversante 13 comme décrit ci-avant, la partie courbe Pb1 du crochet 22 est réalisée par pliage, sensiblement en arc de cercle, du tube 12 qui sera avantageusement choisi initialement sensiblement rectiligne.

De cette façon, à partir d'un tube creux rectiligne avec une percée traversante 13 qui a une section nominale, par exemple de valeur S1, le pliage, notamment en arc de cercle, de la partie de tube qui correspond à la partie courbe Pb1 du crochet, provoque un léger écrasement de cette partie de tube. Cet écrasement a pour conséquence de réduire de façon notable la section de la portion de percée traversante dans cette partie de tube, jusqu'à une valeur S2 qui est inférieure à sa valeur nominale S1.

Quant à la valeur de la section de la portion de percée traversante dans la partie de tube correspondant à la partie rectiligne Pb2 du crochet, elle va repasser progressivement de la valeur S2 qu'elle avait dans la partie courbe du crochet, à sa valeur nominale S1, ce qui a pour effet de donner, à la sortie distale de la percée traversante, une forme évasée comme illustré sur la figure 2, et donc de conférer à cette portion de percée traversante une forme de tronc de cône.

Quant aux moyens 16 pour coupler une source externe d'alimentation en gaz avec l'orifice de sortie 11, ils comportent : un vérin 30 ou analogue, connu en lui-même, comprenant une chambre 31 et une sortie 32 réalisée dans la chambre ; des moyens pour relier la sortie 32 réalisée dans la chambre avec l'orifice de sortie 11 réalisé sur la poignée, par exemple un conduit de sortie dans lequel est montée une soupape anti-retour ou analogue ; un piston 33 monté de façon commandable en translation et de façon étanche dans la chambre 31, pour définir un espace à volume variable ; des moyens 34 pour commander la translation du piston 33 dans la chambre 31, par exemple un bouton moleté couplé au piston par une tige de piston ; et des moyens pour relier de façon fluidique la source externe de gaz avec l'espace à volume variable, par exemple une conduite métallique de liaison à laquelle est aussi couplée une valve anti-retour avec une entrée de connexion du type fluidique.

A titre d'exemple, le gaz qui peut être avantageusement utilisé est du CO₂ sous une pression par exemple comprise entre 1 bar et 10 bars, qui peut être délivré sur l'entrée de connexion.

Des expérimentations ont permis de démontrer qu'un instrument selon l'invention donne de très bons résultats, lorsque la forme en tronc de cône 20 de la portion de percée traversante 13 qui est réalisée dans la portion de tube qui comporte l'extrémité distale 115 présente un angle solide sensiblement égal à 10 degrés.

Il faut noter que cet instrument peut aussi être utilisé comme bistouri électrique pour réaliser des dissections de tissus vivants. Dans ce cas, le tube 12 est en un matériau électriquement conducteur, et l'instrument comporte en outre des moyens 18 pour connecter ce tube à une source d'énergie électrique, par exemple par un connecteur électrique lié au tube 12 via le conduit de sortie, le vérin 30 et la conduite métallique de liaison, ces trois derniers éléments comportant des parties métalliques électriquement conductrices pour réaliser la liaison électrique entre le connecteur électrique et le tube 12. Il est cependant avantageux que l'instrument comporte aussi une gaine électriquement isolante 19 enveloppant extérieurement, au moins partiellement, la poignée de manipulation 10 (Figure 2).

L'utilisation de l'instrument selon l'invention est identique à celle des instruments du même type de l'art antérieur. En conséquence, dans le seul but de simplifier la présente description, elle ne sera pas plus amplement développée ici.

Il sera seulement noté que les expérimentations menées par la Demanderesse avec des instruments selon l'invention tels que celui décrit ci-dessus ont prouvées que les inconvénients attribués aux instruments de l'art antérieur mentionnés au préambule de la présente description, ont presque totalement disparu. En effet : la sortie distale 15 de la percée traversante 13 au niveau de l'extrémité distale 115 du tube 12 ne subit plus d'obturation par divers éléments provenant du sang et l'éjection du gaz, par exemple pour séparer les uns des autres des tissus vivants à disséquer, se fait ainsi sans interruption, ce qui favorise grandement le travail des chirurgiens et, pour le bien des patients, raccourcit la durée des interventions chirurgicales.

## Revendications

1. Instrument pour souffler un gaz dans un organe vivant pour favoriser la dissection de cet organe, comportant :
• une poignée de manipulation (10) apte à être saisie par un chirurgien,
• un orifice de sortie (11) réalisé sur ladite poignée,
• un tube (12) comportant une percée traversante (13) définie entre une entrée proximale (14) et une sortie distale (15), ledit tube étant défini entre une extrémité proximale (114) et une extrémité distale (115), ladite percée traversante (13) étant réalisée dans une partie (Pb) du tube (12) comportant l'extrémité distale (115) se terminant, sur une longueur déterminée vers cette extrémité distale (115), sensiblement en tronc de cône (20), optionnellement de révolution,
• des moyens pour connecter l'entrée proximale (14) de la percée traversante (13) avec ledit orifice de sortie (11) de la poignée (10), et
• des moyens (16) pour coupler une source externe d'alimentation en gaz avec ledit orifice de sortie (11), · la grande base (25) de ce tronc de cône étant sensiblement dans le plan de la sortie distale (15) de la percée traversante, la petite base du tronc de cône ayant le même diamètre que la percée traversante.

2. Instrument selon la revendication 1, **caractérisé par le fait que** la partie (Pb) du tube (12) comportant l'extrémité distale (115) est conformée en crochet (22), ledit crochet comportant une partie courbe (Pb1) suivie d'une partie sensiblement rectiligne (Pb2) qui comprend l'extrémité distale (115).

3. Instrument selon la revendication 2, **caractérisé par le fait que** la portion de percée traversante (13) qui se termine, sur une longueur déterminée vers l'extrémité distale (115), en tronc de cône est réalisée dans la partie sensiblement rectiligne (Pb2) du crochet (22), la petite base (26) dudit tronc de cône étant sensiblement dans le plan qui sépare les parties respectivement rectiligne et courbe (Pb1, Pb2).

4. Instrument selon la revendication 3, **caractérisé par le fait que** la partie (24) du tube comprise entre l'orifice de sortie (11) réalisé sur la poignée (10) et la partie courbe (Pb1) du crochet est un tube cylindrique sensiblement rectiligne optionnellement de révolution.

5. Instrument selon l'une des revendications 2 à 4, **caractérisé par le fait que** la partie courbe (Pb1) du crochet (22) est réalisée par pliage sensiblement en arc de cercle du dit tube.

6. Instrument selon l'une des revendications précédentes, **caractérisé par le fait que** les moyens (16) pour coupler une source d'alimentation externe en gaz avec ledit orifice de sortie (11) comportent :
• un vérin (30) comprenant une chambre (31) et une sortie (32) réalisée dans ladite chambre,
• des moyens pour relier ladite sortie (32) réalisée dans ladite chambre avec l'orifice de sortie (11) réalisé sur la poignée,
• un piston (33) monté de façon commandable en translation et de façon étanche dans ladite chambre (31), pour définir un espace à volume variable,
• des moyens (34) pour commander la translation du dit piston (33) dans ladite chambre (31), et
• des moyens pour relier de façon fluidique une source de gaz externe avec ledit espace à volume variable.

7. Instrument selon l'une des revendications précédentes, **caractérisé par le fait que** ledit gaz est du CO₂ sous une pression comprise entre 1 bar et 10 bars.

8. Instrument selon l'une des revendications précédentes, **caractérisé par le fait que** ledit tronc de cône présente un angle solide sensiblement égal à 10 degrés.

9. Instrument selon l'une des revendications précédentes, **caractérisé par le fait que** ledit tube (12) est en un matériau électriquement conducteur, et qu'il comporte des moyens (18) pour connecter ledit tube à une source d'énergie électrique et une gaine électriquement isolante (19) enveloppant extérieurement au moins partiellement ladite poignée de manipulation (10).

## Patentansprüche

1. Instrument zum Einblasen eines Gases in ein lebendes Organ, um die Sezierung dieses Organs zu begünstigen, umfassend:
• einen Bediengriff (10), der von einem Chirurgen erfasst werden kann,
• eine Ausgangsöffnung (11), die an diesem Griff ausgeführt ist,
• ein Rohr (12), das einen durchgehenden Durchlass (13) umfasst, der zwischen einem proximalen Eingang (14) und einem distalen Ausgang (15) definiert ist, wobei das Rohr zwischen einem proximalen Ende (114) und einem distalen Ende (115) definiert ist, wobei der durchgehende Durchlass (13) in einem Teil (Pb) des Rohrs (12) ausgeführt ist, der das distale Ende (115) umfasst, das über eine bestimmte Länge zu diesem distalen Ende (115) hin im Wesentlichen in einem Kegelstumpf (20), optional einem Rotationskegelstumpf, endet,
• Mittel, um den proximalen Eingang (14) des durchgehenden Durchlasses (13) mit der Ausgangsöffnung (11) des Griffs (10) zu verbinden, und
• Mittel (16) zum Ankoppeln einer externen Gaszuführquelle an die Ausgangsöffnung (11), wobei die große Grundfläche (25) des Kegelstumpfes im Wesentlichen in der Ebene des distalen Ausgangs (15) des durchgehenden Durchlasses liegt und die kleine Grundfläche des Kegelstumpfes den gleichen Durchmesser wie der durchgehende Durchlass hat.

2. Instrument nach Anspruch 1, **dadurch gekennzeichnet, dass** der das distale Ende (115) umfassende Teil (Pb) des Rohrs (12) als Haken (22) geformt ist, wobei der Haken einen gekrümmten Teil (Pb1) umfasst, gefolgt von einem im Wesentlichen geradlinigen Teil (Pb2), der das distale Ende (115) umfasst.

3. Instrument nach Anspruch 2, **dadurch gekennzeichnet, dass** der Abschnitt des durchgehenden Durchlasses (13), der über eine bestimmte Länge zum distalen Ende (115) hin in einem Kegelstumpf endet, in dem im Wesentlichen geradlinigen Teil (Pb2) des Hakens (22) ausgebildet ist, wobei die kleine Grundfläche (26) des Kegelstumpfes im Wesentlichen in der Ebene liegt, die den jeweils geradlinigen und gekrümmten Teil (Pb1, Pb2) voneinander trennt.

4. Instrument nach Anspruch 3, **dadurch gekennzeichnet, dass** der Teil (24) des Rohrs zwischen der am Griff (10) ausgebildeten Ausgangsöffnung (11) und dem gekrümmten Teil (Pb1) des Hakens ein im Wesentlichen geradliniges zylindrisches, optional rotationssymmetrisches Rohr ist.

5. Instrument nach einem der Ansprüche 2 bis 4, **dadurch gekennzeichnet, dass** der gekrümmte Teil (Pb1) des Hakens (22) durch im Wesentlichen bogenförmiges Krümmen des Rohrs hergestellt wird.

6. Instrument nach einem der vorstehenden Ansprüche, **dadurch gekennzeichnet, dass** die Mittel (16) zum Ankoppeln einer externen Gaszuführquelle an die Ausgangsöffnung (11) umfassen:
• einen Zylinder (30), der eine Kammer (31) und einen in der Kammer ausgebildeten Ausgang (32) umfasst,
• Mittel zum Verbinden des in der Kammer ausgebildeten Ausgangs (32) mit der am Griff ausgebildeten Ausgangsöffnung (11),
• einen Kolben (33), der verschiebbar und dicht in der Kammer (31) montiert ist, um einen Raum mit variablem Volumen zu definieren,
• Mittel (34), um die Verschiebung des Kolbens (33) in der Kammer (31) zu steuern und
• Mittel, um eine externe Gasquelle mit dem Raum mit variablem Volumen strömungstechnisch zu verbinden.

7. Instrument nach einem der vorstehenden Ansprüche, **dadurch gekennzeichnet, dass** es sich bei dem Gas um CO₂ unter einem Druck zwischen 1 bar und 10 bar handelt.

8. Instrument nach einem der vorstehenden Ansprüche, **dadurch gekennzeichnet, dass** der Kegelstumpf einen Raumwinkel von im Wesentlichen 10 Grad aufweist.

9. Instrument nach einem der vorstehenden Ansprüche, **dadurch gekennzeichnet, dass** das Rohr (12) aus einem elektrisch leitenden Material besteht und Mittel (18) zum Verbinden des Rohrs mit einer Stromquelle und eine elektrisch isolierende Hülle (19) umfasst, die den Bediengriff (10) zumindest teilweise äußerlich umhüllt.

## Claims

1. Instrument for blowing a gas into a live organ to aid the dissection of that organ, comprising:
• a control handle (10) capable of being grasped by a surgeon,
• an outlet orifice (11) made on said handle,
• a tube (12) comprising a through-hole (13) defined between a proximal inlet (14) and a distal outlet (15), said tube being defined between a proximal end (114) and a distal end (115), said through-hole (13) being made in a part (Pb) of the tube (12) comprising the distal end (115) ending, over a determined length towards this distal end (115), substantially in the shape of a truncated cone (20), optionally of revolution,
• means for connecting the proximal inlet (14) of the through-bore (13) with said outlet port (11) of the handle (10), and
• means (16) for coupling an external gas supply source with said outlet orifice (11), the large base (25) of this truncated cone being substantially in the plane of the distal outlet (15) of the through-hole, the small base of the truncated cone having the same diameter as the through-hole.

2. Instrument according to claim 1, **characterised in that** the part (Pb) of the tube (12) comprising the distal end (115) is shaped as a hook (22), said hook comprising a curved part (Pb1) followed by a substantially rectilinear part (Pb2) which comprises the distal end (115).

3. Instrument according to claim 2, **characterised in that** the through-hole portion (13) which terminates, over a determined length towards the distal end (115), in a truncated cone is made in the substantially rectilinear part (Pb2) of the hook (22), the small base (26) of said truncated cone being substantially in the plane which separates the respectively rectilinear and curved parts (Pb1, Pb2).

4. Instrument according to claim 3, **characterised in that** the part (24) of the tube between the outlet orifice (11) made on the handle (10) and the curved part (Pb1) of the hook is a substantially rectilinear cylindrical tube optionally of revolution.

5. Instrument according to any one of claims 2 to 4, **characterised in that** the curved part (Pb1) of the hook (22) is produced by folding said tube substantially in a semi-circle.

6. Instrument according to any one of the preceding claims, **characterised in that** the means (16) for coupling an external gas supply source with said outlet orifice (11) comprise:
• a cylinder (30) comprising a chamber (31) and an outlet (32) made in said chamber,
• means for connecting said outlet (32) made in said chamber with the outlet orifice (11) made on the handle,
• a piston (33) mounted in a translationally controllable and sealed manner in said chamber (31), to define a variable volume space,
• a means (34) for controlling the translation of said piston (33) in said chamber (31), and
• means for fluidly connecting an external gas source with said variable volume space.

7. Instrument according to any one of the preceding claims, **characterised in that** said gas is CO2 at a pressure of between 1 bar and 10 bar.

8. Instrument according to any one of the preceding claims, **characterised in that** said truncated cone has a solid angle substantially equal to 10 degrees.

9. Instrument according to any one of the preceding claims, **characterised in that** said tube (12) is made of an electrically conductive material, and **in that** it comprises means (18) for connecting said tube to a source of electrical energy and an electrically insulating sheath (19) at least partially externally enveloping said control handle (10).
